(19) Europäisches Patentamt
European Patent Office
Office européen des brevets

(11) Publication number: 0 000 257
B1

(12) **EUROPEAN PATENT SPECIFICATION**

(45) Date of publication of patent specification: 17.02.82

(51) Int. Cl.[3]: C 07 D 311/76, C 11 B 9/00

(21) Application number: 78300056.5

(22) Date of filing: 20.06.78

(54) **Benzo(c)pyran derivatives, process for their preparation and their use in perfumery.**

(30) Priority: 21.06.77 GB 2586377
05.04.78 GB 1333878

(43) Date of publication of application:
10.01.79 Bulletin 79/1

(45) Publication of the grant of the European patent:
17.02.82 Bulletin 82/7

(84) Designated Contracting States:
BE CH DE FR GB NL SE

(56) References cited:
US - A - 2 483 824

(73) Proprietor: BUSH BOAKE ALLEN Limited
Blackhorse Lane Walthamstow
London E17 5QP (GB)

(72) Inventor: Cookson, Richard Clive
Manor Farm, Stratford Tony
Wiltshire (GB)
Inventor: Ferro, Lorenzo
Apartment 319, 67 East 11th Street
New York 10,003 (US)
Inventor: Ferber, Gerald John
18 Sherwood Road, Barkingside
Ilford, Essex (GB)

(74) Representative: Bloxam, George Arthur
c/o Albright & Wilson Limited 1, Knightsbridge
Green
London SW1X 7QD (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 000 257 B1

Courier Press, Leamington Spa, England.

Benzo (c) pyran derivatives, process for their preparation and their use in perfumery

This invention is concerned with certain novel organic compounds and with perfumery compositions containing the compounds. It has been discovered that these compounds exhibit unique, attractive odours which render them useful in ingredients of perfumery compositions which compositions find use in a wide variety of applications.

From one aspect our invention provides compounds of the formula:

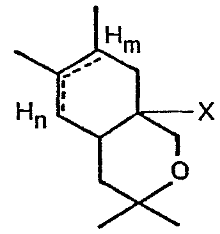

wherein X represents a hydrogen atom or an alkyl group having from 1 to 4 carbon atoms and m has a value 0 or 1, n has a value 1 or 2 and n + m = 2 the dashed lines indicating alternative positions for a unit of unsaturation. Such compounds may be individual steroisomers or mixtures of the possible steroisomers of compounds having the above formula.

These compounds have been discovered to have a fresh floral type of odour, with a blend of prominent neroli, bitter orange notes. Furthermore, the odours of these compounds blend harmoniously with those of other known odoriferous chemicals to produce useful compounded perfumery compositions.

The novel compounds of this invention are derivatives of 3,3,7,8-tetramethyl-2-oxabicyclo-(4,4,0)-decane which will hereinafter be referred to for convenience as bigarade-oxides. They may be employed as a major ingredient of these compositions depending upon the desired overall odour required. In general the bigarde-oxides will constitute from 2 to 15, preferably 3 to 7% by weight of the composition. The perfumery compositions of this invention may find use as such or after dilution, but more usually they are added in small proportions to other materials such as space sprays or to soap cosmetic or deodorant compositions or to substrates such as fibre fabric or paper products in order to provide them with agreeable olfactory properties. Such compositions are products of commerce and they may comprise a simple or complex mixture of individual perfumery compounds.

Thus, from a second aspect our invention provides a compounded perfumery composition comprising a plurality of odoriferous chemicals together with at least one compound having the formula:—

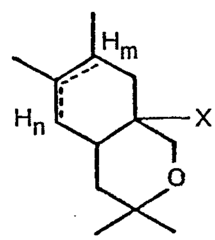

wherein X and m and n are as hereinafter defined.

The unique fruity floral odours of the novel bigarade-oxides of our invention find special use in compositions designed for use in various perfumed bath preparations such as bath salts etc.

These novel perfumery compositions may be compounded according to recognised techniques of the perfumery arts employing known odoriferous perfumery ingredients such as those described in the standard textbooks of the art, e.g. "Soap, Perfumery and Cosmetics" by W.A. Poucher, 8th Edition, published by Chapman and Hall (London) 1974; "Perfume and Flavour Chemicals" by S. Arctander published by the author (Montclair) 1969 "Perfume and Flavour Materials of Natural Origin" also by S. Arctander self-published Elizabeth New Jersey (1960) and "Perfume Technology" by M. Billot and F.V. Wells published by Ellis Horwood Ltd. 1975. Specific odoriferous ingredients which may be blended with the bigarade-oxides in a compounded perfumery composition are the derivatives of 2,6-dimethyl-2-alkoxy octan-7-ol (as claimed in our Dutch Patent Application No. 72. 15238), vetivert oil, vetiverol, vetiveryl acetate guaic wood oil, esters of anthranilic acid such as the methyl, N methyl methyl, ethyl, phenyl-ethyl, cinnamyl, linalyl, methyl and geranyl esters, benzyl acetate, lemon oil, dimethyl benzyl carbinol, dimethyl benzyl carbinyl acetate, rose absolute, jasmin absolute, ionones, isononyl acetate, methyl phenyl acetate, styrallyl acetate, B. phenyl ethanol, citronellol, citronellal, hydroxy citronellal, geranium oil, geraniol, linalol, nerol, lavandin oil, linalyl acetate, patchouli oil, petitgrain oil, bergamot

oil, heliotropin, ethylene rassylate, undecyl aldehyde, cinnamaldehyde, benzyl-salicylate, cinnamyl alcohol, clove bud oil, bay oil, nutmeg oil, pimento berry oil, terpineol, ylang oil, benzyl benzoate, sandal-wood oil, clary sage oil, amyl salicylate, labdanum resin, methyl ionones, dihydro-myrcenol, orange oil, vanillin, ethylvanillin, olibanum resin, musk ambrette, rhodinol, mandarin oil, methylnonyl acetaldehyde, neroli oil, cedrol, oakmoss, isovalanone, eugenol, iso-eugenol, cedarwood oil, p-tert-butyl cyclo-hexyl acetate.

Typically, the novel bigarade-oxides are blended with at least two, usually at least five and preferably at least ten of the foregoing ingredients.

Preferred compounds for present use are those wherein X represents a methyl group or a hydrogen atom. Most preferably the isomers wherein n and m have a value of 1 are used.

Particularly preferred odoriferous ingredients for blending with the bigarade-oxides are linalol, linalylacetate, bergamot oil, grapefruit oil, lemon oil, orange oil, petitgrain oil, hexylcinnamic aldehyde, benzylsalicylate, methyl ionones, 2-alkoxy-2,6 dimethyl-octan-7-ols, methylanthranilate, geraniol and nerol and esters thereof, neroli oil, farnesol, nerolidol, eugenol, isoeugenol, patchouli oil, vetiveryl acetate, cedryl acetate, p-tertiary butyl cyclohexyl acetate and terpineol.

The novel bigarade-oxides may conveniently be made by a multi-step synthesis which uses the triene known as allo ocimene as its starting material. Allo acimene is readily available as a product of the thermal isomerisation of ocimene, a triene found as constituent of several essential oils or more usually from the thermal isomerisation of a pinene.

As a first step in this synthesis allo-ocimene is reacted with an unsaturated dienophile having the formula: $CH_2=CXY$ the reaction being an example of Diels-Alder addition. The reaction proceeds according to the following equation:—

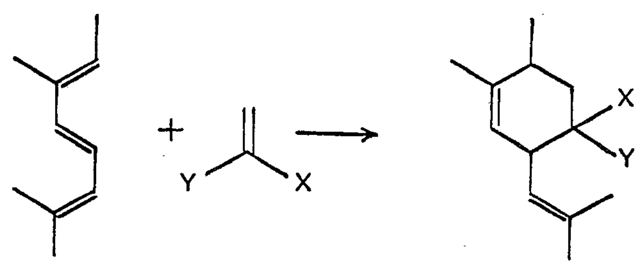

The desired intermediate for the production of the novel compounds of the invention is the compound of formula:

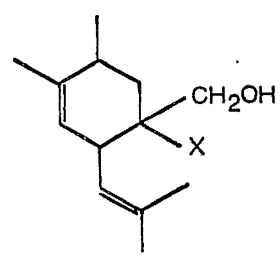

Which can be produced directly by using an unsaturated alcohol as the dienophile in the above reaction, e.g. where X represents a hydrogen atom allyl alcohol can be used. Other dienophiles which yield adducts which can be converted to an alcohol having the above formula may also be employed and the nature of the substituent Y will vary accordingly. Thus acrolein, acrylic acid, acrylate esters and acrylic acid chloride can be employed. Preferably, an unsaturated aldehyde is employed as the dienophile because of its properties as a dienophile and the ease with which the adduct aldehyde can be converted to the desired alcohol intermediate. Thus in this case of the preferred compounds where X represents a hydrogen atom acrolein is added to allo-ocimene. The Diels Alder addition can be carried out at elevated temperatures and pressures e.g. 120 to 180°C and 3.51 to 10.53 $Kg/cm^2$ or at ambient temperatures in the presence of a suitable electrophilic catalyst such as aluminium chloride. The Diels-Alder addition of acrolein and allyl alcohol to allo-ocimene has been reported in the Journal of the Chemical Society of Japan Volume 5 (1973 pages 1064 to 1066). This disclosure describes the addition to a mixture of a trans-trans and trans-cis allo-ocimene. When using a mixture of these two stereoisomers we prefer to carry out the reaction under elevated temperatures and pressures. When the low temperature catalysed reaction is employed the trans-cis isomer reacts extremely slowly. The Diels-Alder addition is probably sterospecific and the product which is preferably separated by fractional distillation comprises a mixture of the four steroisomeric adducts having the above formula. These adducts are then converted to the corresponding alcohols. In the preferred case the aldehydes are reduced using the conventional technique of synthetic organic chemistry. Where other dienophiles are employed in the reaction step such as an unsaturated carboxylic acid, carboxylic acid ester and

carboxylic acid chloride the adducts are likewise converted to the corresponding alcohols using conventional techniques.

The conversion of the aldehyde to the corresponding alcohol may conveniently be achieved using catalytic hydrogenation i.e. heating the aldehyde adduct(s) at an elevated temperature of from 150°C to 170°C under superatmospheric pressure say 12.66 to 14.07 $Kg/cm^2$ in an atmosphere of hydrogen gas and in the presence of a suitable catalyst. Preferably, the catalyst employed is copper chromite but other conventional catalysts such as nickel, copper and palladium may be employed. Alternatively, the reduction can be achieved using chemical methods in particular reduction with metal hydrides such as lithium aluminium hydride or sodium borohydride which reagents are normally added to the aldehyde as a solution in ether or water respectively, the reaction proceeding smoothly at ambient temperature. In conducting this reduction care should be taken to ensure that the ethylenic units of unsaturation present in the molecule are not attacked. The alcohols may be separated from the mixture of products formed using conventional techniques e.g. fractional distillation or used directly in the next stage in the synthesis.

The alcohol thus obtained may be cyclised to the bigarade-oxide by heating preferably under reflux in the presence of a protonic acid catalyst, conveniently an aqueous solution of a mineral acid or phosphoric acid or an organic solution of a sulphonic acid. The reaction will usually go to completion under reflux with a period of a few hours e.g. 6 to 20 hours. Preferably an aqueous acid solution is employed as the catalyst. In order to speed the reaction a relatively concentrated solution e.g. 15 to 35% by weight is preferably employed in such a quantity that the volume of the aqueous phase is at least equal to and preferably at least twice the volume of the organic phase. The use of more concentrated acid solutions gives a desirably high yield in a relatively short time e.g. 6 to 10 hours. The oil layer may be separated from the aqueous layer and fractionated to give the desired bigarade-oxide product.

The use of organic solutions of sulphonic acids e.g. para-toluene sulphonic acids in benzene or in an alkyl benzene speeds the reaction and favours the production of these novel compounds wherein n and m have a value of 1. The organic layer is preferably washed with a dilute aqueous solution of a caustic alkali and the bigarade-oxides are then separated by fractional distillation.

The product normally comprises a mixture of the various steroisomers of compounds having the appropriate molecular formula as defined above. It will normally comprise a mixture of the compounds wherein n has a value of 1 with those wherein n has a value of 2. This mixture can be fractionated to separate those compounds having the above formula wherein $n = 1$ from these where $n = 2$. In the former the novel odoriferous character is more pronounced. However, the presence of the latter complements this odour in a desirable manner and preferably this separation step is omitted. Formation of the compounds wherein n has a value of 2 is favoured by the treatment of the alcohol with acid for a longer period or by using a stronger acid.

The invention is illustrated by the following examples:—

EXAMPLE 1

Preparation of 1,6-dimethyl-3-isobutenyl-4-formyl cyclohexene

544 g of allo-ocimene (85% 4E, 6Z; 15% 4E, 6E) and 224 g of acrolein were placed in a stainless steel vessel which is equipped to withstand a high internal pressure at elevated temperatures. 1 gm of embanox, a polymerisation inhibitor, was added. The air in the vessel was displaced with nitrogen and the vessel was closed. The temperature was raised to 165—170°C and maintained in the range for 6 hours. The maximum pressure recorded was 10.53 $Kg/cm^2$. The reaction mixture was then distilled and a mixture of four isomeric aldehydes collected which represented a 70% yield of the desired product. The percentages of adducts were determined by GLC to be as follows:

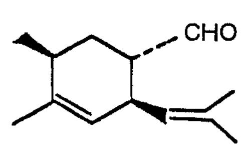

A 4%

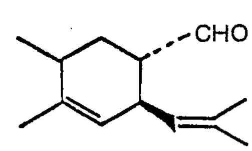

B 11%

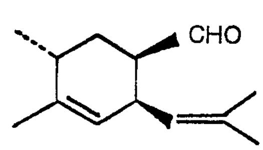

C 72%

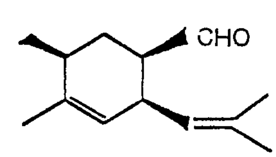

D 13%

## EXAMPLE 2

Preparation of 1,6-dimethyl-3-isobutenyl-4-formyl-cyclohexene.

408 g of allo-ocimene (85% 4E 6Z, 15% 4E 6E) and 202 g of acrolein were placed in a stainless steel vessel which is equipped to withstand a high internal pressure at elevated temperatures. 1 gm of hydroquinone, a polymerisation inhibitor, was added. The air in the vessel was displaced with nitrogen and the vessel closed. The temperature was raised to 145—150°C and maintained in the range for 7.5 hours. The maximum pressure recorded was 7.39 $Kg/cm^2$. The reaction mixture was then distilled and a mixture of four aldehydes collected which represented a 66% yield of the desired product. The percentages of adduts were A 4%, B 11%, C 74% and D 11% where the letters A, B, C, D represent the isomer denoted as such in Example 1.

## EXAMPLE 3

Preparation of 1,6-dimethyl-3-isobutenyl-4-hydroxy-methylcyclohexene.

400 gm of the mixed aldehyde product obtained from 1 above was mixed with 200 mls of methanol and 8 gms of copper chromite and shaken in an atmosphere of hydrogen at a temperature of 180°C and a pressure of 12.66 to 14.07 $Kg/cm^2$ for a period of six hours. The product was filtered and distilled to give a mixture of four isomeric alcohols in 85% yield.

## EXAMPLE 4

Preparation of 1,6-dimethyl-3-isobutenyl-4-hydroxymethyl cyclohexene.

443 g of the mixed aldehyde product obtained from 2 above was mixed with 5 g copper chromite and shaken in an atmosphere of hydrogen at a temperature of 150°C and a pressure of 9.13 to 10.53 $Kg/cm^2$ for 14 hours. The product was filtered and distilled to give a mixture of four isomeric alcohols in 85% yield.

## EXAMPLE 5

Preparation of 3,3,7,8-tetramethyl-2-oxabicyclo-[4,4,0] dec-6-en.

1500 mls of a 10% aqueous solution of phosphoric acid were added to 500 gm of the alcohol mixture obtained in (3) above and the solution refluxed gently with stirring for a period of 8 hours. The oil layer was separated and washed with dilute aqueous caustic alkali, dried over magnesium sulphate and distilled to yield the desired product.

## EXAMPLE 6

Preparation of 3,3,7,8-tetramethyl-2-oxabicyclo-[4,4,0] dec-6-enes and 3,3,7,8-tetramethyl-2-oxabicyclo-[4,4,0] dec-7-enes.

3000 mls of a 30% aqueous solution of phosphoric acid were added to 1160 g of the alcohol mixture obtained in 3 above and the solution refluxed gently with stirring for a period of 12 hours. The oil layer was separated and washed with dilute aqueous caustic alkali and then water. The crude product was distilled to give a 74% yield of the six possible oxide isomers.

## EXAMPLE 7

Preparation of 3,3,7,8-tetramethyl-2-oxabicyclo-[4,4,0] dec-6-enes and 3,3,7,8-tetramethyl-2-oxabicyclo-[4,4,0] dec-7-enes.

20 g of para-toluene sulphonic acid in 384 g of ethylbenzene were added to 384 g of the alcohol mixture obtained in 3 above and the solution was stirred at 70—80°C for 7.5 hr. The reaction mixture was washed with dilute aqueous caustic alkali and then water. The crude product was distilled to give a 77% yield of the six possible oxide isomers.

EXAMPLE 8

A compounded perfumery composition was made up as follows (all parts by weight):—

| | |
|---|---|
| Bergamot oil | 20 |
| B pinene | 100 |
| Limonene | 140 |
| Trans-ocimene | 20 |
| Linalol | 300 |
| Linalylacetate | 80 |
| Petitgrain oil | 40 |
| Terpineol | 30 |
| Geraniol | 30 |
| Nerol | 10 |
| Neryl acetate | 20 |
| Geranyl acetate | 30 |
| Nerolidol | 50 |
| Farnesol | 20 |
| Indole (10% solution in linalol) | 25 |
| Bigarade-oxide (Product of Example 5) | 80 |
| | 1000 |

**Claims**

1. A compound having the formula:—

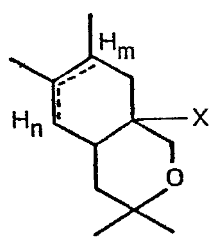

wherein X represents a hydrogen atom or an alkyl group having from 1 to 4 carbon atoms, m has a value of 0 or 1, n has a value of 1 or 2, n + m = 2 and the dashed line indicates alternative positions for a unit of unsaturation.

2. A compound according to claim 1, wherein X represents a methyl group.

3. A compound according to claim 1, wherein X represents a hydrogen atom.

4. A compound according to any of the preceding claims, wherein n and m each have a value of 1.

5. A compounded perfumery composition which comprises a plurality of odoriferous ingredients characterised in that it further comprises at least one compound having the formula

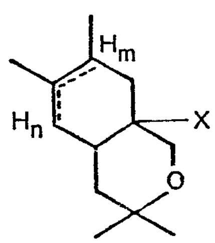

wherein X represents a hydrogen atom or an alkyl group having from 1 to 4 carbon atoms, m has a value of 0 or 1, n has a value of 1 or 2, n + m = 2 and the dashed line indicates alternative positions for a unit of unsaturation.

6. A composition according to claim 5 characterised in that X represents a hydrogen atom.

7. A composition according to either of claims 5 or 6, characterised in that n and m each have a value of 1.

8. A composition according to any of claims 5, 6 and 7 characterised in that the composition further comprises one or more odoriferous chemicals selected from linalol, linalylacetate, bergamot oil, grapefruit oil, lemon oil, orange oil, petitgrain oil, hexylcinnamic aldehyde, benzylsalicylate, methyl ionones, 2-alkoxy-2,6-dimethyl-octan-7-ols, methylanthranilate, geraniol and nerol and esters thereof,

neroli oil, farnesol nerolidol, eugenol, isoeugenol, patchouli oil, vetiveryl acetate, cedryl acetate, p tertiary butyl cyclohexyl acetate and terpineol.

9. A composition according to claim 8, characterised in that the compounds of the formula according to claim 1 comprise from 2 to 15% by weight thereof.

10. A composition according to claim 9, characterised in that the compounds of the formula according to claim 1 comprise from 3 to 7% by weight thereof.

11. A process for the preparation of a compound having the formula:

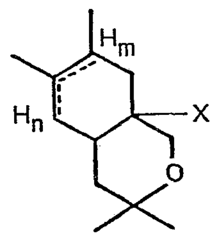

wherein X represents a hydrogen atom or an alkyl group having from 1 to 4 carbon atoms, m has a value of 0 or 1, n has a value of 1 or 2, n + m = 2 and the dashed line indicates alternative positions for a unit of unsaturation characterised in that an alcohol having the formula:

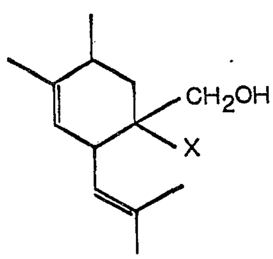

is cyclised by heating in the presence of an acid.

12. A process according to claim 11, characterised in that the acid is phosphoric acid.

13. A process according to claim 12, characterised in that an aqueous acid solution comprising 15 to 35% by weight of acid is employed in a volume at least equal to that of the organic phase.

14. A process according to claim 11, characterised in that the acid is present in the form of a solution of a sulphonic acid in an organic solvent.

15. A process according to claim 14, wherein the acid is para toluene sulphonic acid.

**Revendications**

1. Composé répondant à la formule:

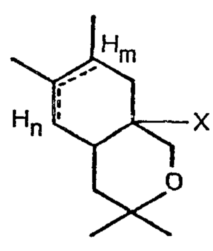

dans laquelle X représente un atome d'hydrogène ou un groupe alkyle comptant 1 à 4 atomes de carbone, *m* possède une valeur de 0 ou 1, *n* une valeur de 1 ou 2, n + m = 2 et la ligne en pointillé indique des positions alternatives pour une unité d'insaturation.

2. Composé selon revendication 1, caractérisé en ce que X représente un groupe méthyle.

3. Composé selon la revendication 1, caractérisé en ce que X représente un atome d'hydrogène

4. Composé selon l'une quelconque des revendications 1 à 3, caractérisé en ce que *n* et *m* possèdent chacun une valeur de 1.

5. Composition pour parfumerie comprenant une pluralité d'ingrédients odoriférants, caractérisée en qu'elle comprend en outre au moins un composé répondant à la formule:

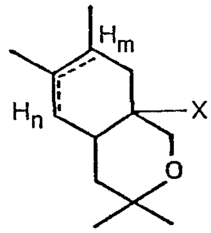

dans laquelle X représente un atome d'hydrogène ou un groupe alkyle ayant 1 à 4 atomes de carbone, *m* possède une valeur de 0 ou 1, *n* une valeur de 1 ou 2, n + m = 2 et la ligne en pointillé indique des positions alternatives pour une unité d'insaturation.

6. Composition selon la revendication 5, caractérisée en ce que X représente un atome d'hydrogène.

7. Composition selon l'une des revendications 5 ou 6, caractérisée en ce que *n* et *m* possèdent chacun une valeur de 1.

8. Composition selon l'une quelconque des revendications 5, 6 et 7, caractérisée en ce que la composition comprend en outre un ou plusieurs agents chimiques odoriférants choisis parmi le linalol, l'acétate de linalyle, l'essence de bergamote, l'essence de pamplemousse, l'essence de citron, l'essence d'orange, l'essanec de petitgrain, l'aldéhyde hexylcinnamique, le salicylate de benzyle, les méthylionones, les 2-alcoxy-2,6-diméthyl-octan-ols, l'anthranilate de méthyle, le géraniol et le nérol ainsi que leurs esters, l'essence de néroli, le farnesol nerolidol, l'eugenol, l'isoeugénol, l'essence de patchouli, l'acétate de vétiveryle, l'acétate de cédryle, le cyclohexylacétate de p-tertio-butyle et et le terpineol.

9. Composition selon la revendication 8, caractérisée en ce que les composés répondant à la formule de la revendication 1 représentent 2 à 15% en poids de celle-ci.

10. Composition selon la revendication 9, caractérisée en ce que les composés répondant à la formule de la revendication 1 représentent 3 à 7% en poids de celle-ci.

11. Procédé pour l'obtention d'un composé répondant à la formule:

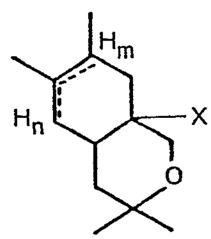

dans laquelle X représente un atome d'hydrogène ou un groupe alkyle ayant 1 à 4 atomes de carbone, *m* possède une valeur de 0 ou 1, *n* une valeur de 1 ou 2, n + m = 2 et la ligne en pointillé indique des positions alternatives pour une unité d'insaturation, caractérisé en ce qu'on cyclise un alcool de formule:

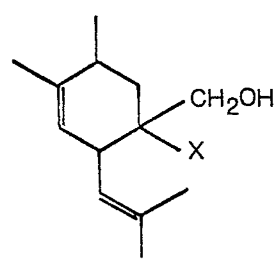

par chauffage en présence d'un acide.

12. Procédé selon la revendication 11, caractérisé en ce que l'acide est l'acide phosphorique.

13. Procédé selon la revendication 12, caractérisé en ce qu'une solution acide aqueuse comprenant 15 à 35% en poids d'acide est utilisée en un volume au moins égal à celui de la phase organique.

14. Procédé selon la revendication 11, caractérisé en ce que l'acide est présent sous la forme d'une solution d'un acide sulfonique dans un solvant organique.

15. Procédé selon la revendication 14, caractérisé en ce que l'acide est l'acide para toluène-sulfonique.

**Patentansprüche**

1. Verbindung der Formel

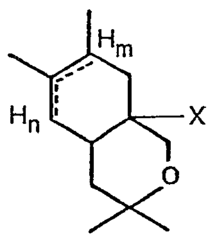

in der X ein Wasserstoffatom oder eine Alkylgruppe mit 1 bis 4 Kohlenstoffatomen bedeutet, m einen Wert von 0 oder 1 hat, n einen Wert von 1 oder 2 hat, n + m = 2 und die gestrichelte Linie alternative Stellungen für eine ungesättigte Bindung anzeigt.

2. Verbindung nach Anspruch 1, in der X eine Methylgruppe darstellt.

3. Verbindung nach Anspruch 1, in der X ein Wasserstoffatom darstellt.

4. Verbindung nach jedes der vorhergehenden Ansprüche, in der n und m jeweils einen Wert 1 haben.

5. Zusammengesetzte Parfümkompositionen, enthaltend eine Mehrzahl riechender Bestandteile, dadurch gekennzeichnet, daß sie außerdem wenigstens eine Verbindung der Formel

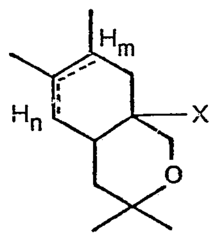

enthält, in der X ein Wasserstoffatom oder eine Alkylgruppe mit 1 bis 4 Kohlenstoffatomen bedeutet, m einen Wert von 0 oder 1 hat, n einen Wert von 1 oder 2 hat, n + m = 2 und die gestrichelte Linie alternative Stellungen für eine ungesättigte Bindung anzeigt.

6. Komposition nach Anspruch 5, dadurch gekennzeichnet, daß X ein Wasserstoffatom bedeutet.

7. Komposition nach Anspruch 5 oder 6, dadurch gekennzeichnet, daß n und m jeweils einen Wert 1 haben.

8. Komposition nach jedem Ansprüche 5, 6 oder 7, dadurch gekennzeichnet, daß sie außerdem eine oder mehrere riechende chemische Verbindungen ausgewählt aus Linalol, Linalylacetat, Bergamotteöl, Pampelmusenöl, Zitronenöl, Orangenöl, Petitgrainöl, Hexylzimtsäurealdehyd, Benzylsalicylat, Methylionone, 2-Alkoxy-2,6-dimethyl-octan-7-ole, Methylanthranilat, Geraniol und Nerol und deren Ester Neroliöl, Farnesol, Nerolidol, Eugenol, Isoeugenol, Patchouliöl, Vetiverylacetat, Cedrylacetat, p-tertiär-Butylcyclohexylacetat und Terpineol enthält.

9. Komposition nach Anspruch 8, dadurch gekennzeichnet, daß die Verbindungen der Formel nach Anspruch 1 2 bis 15 Gew.-% der Gesamtkomposition ausmachen.

10. Komposition nach Anspruch 9, dadurch gekennzeichnet, daß die Verbindungen der Formel gemäß Anspruch 1 3 bis 7 Gew.-% der Gesamtkomposition ausmachen.

11. Verfahren zur Herstellung einer Verbindung der Formel

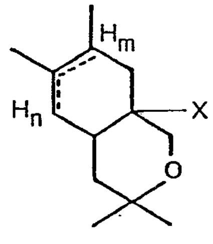

in der X ein Wasserstoffatom oder eine Alkylgruppe mit 1 bis 4 Kohlenstoffatomen bedeutet, n einen Wert 0 oder 1 hat, n einer Wert 1 oder 2 hat, n + m = 2 und die gestrichelte Linie alternative Stellungen für eine ungesättigte Bindung anzeigt, dadurch gekennzeichnet, daß ein Alkohol der Formel

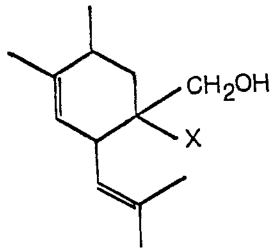

durch Erhitzen in Gegenwart einer Säure cyclisiert wird.

12. Verfahren nach Anspruch 11, dadurch gekennzeichnet, daß die Säure Phosphorsäure ist.

13. Verfahren nach Anspruch 12, dadurch gekennzeichnet, daß eine wäßrige Säurelösung, die 15 bis 35 Gew.-% Säure enthält, in einem Volumen angewendet wird, das wenigstens gleich dem Volumen der organischen Phase ist.

14. Verfahren nach Anspruch 11, dadurch gekennzeichnet, daß die Säure in Form einer Lösung einer Sulfonsäure in einem organischem Lösungsmittel vorliegt.

15. Verfahren nach Anspruch 14, dadurch gekennzeichnet, daß die Säure p-Toluolsulfonsäure ist.